# EUROPEAN PATENT APPLICATION

(11) **EP 4 480 389 A1**
(43) Date of publication of application: **25.12.2024**
(21) Application number: 23770669.2
(22) Date of filing: 10.03.2023
(51) Int. Cl.: A61B 3/103

(54) **OPHTHALMIC DEVICE AND CONTROL METHOD THEREOF**

(30) Priority: 18.03.2022 JP 2022044031
(71) Applicant: TOPCON CORPORATION, Tokyo 174-8580 (JP)
(72) Inventor: YUKIMORI, Takafumi, Tokyo 174-8580 (JP); NAKANISHI, Hajime, Tokyo 174-8580 (JP)
(74) Representative: Schmitt-Nilson Schraud Waibel Wohlfrom Patentanwälte Partnerschaft mbB
(86) International application number: PCT/JP2023/009247
(87) International publication number: WO 2023/176714

(57) **Abstract**

An ophthalmic device and a control method thereof are provided which can acquire an ocular characteristic of a subject eye simply and in a short period of time even when the subject eye has nystagmus or the subject is a child. The ophthalmic device includes a detecting unit configured to detect a relative position of a subject eye relative to a ocular characteristic acquiring unit, a provisional alignment controlling unit configured to drive a relatively-moving unit based on a detection result by the detecting unit and perform provisional alignment of the ocular characteristic acquiring unit relative to the subject eye, a re-detecting unit configured to repeatedly detect the relative position by the detecting unit after completion of the provisional alignment, a determining unit configured to determine whether the ocular characteristic acquiring unit has been brought closer to the subject eye by a distance greater than or equal to a predetermined threshold value by the provisional alignment based on the detection result by the detecting unit before and after the provisional alignment, and an operation mode selecting unit configured to select either one of a normal mode and a quick mode as an operation mode for alignment of the ocular characteristic acquiring unit relative to the subject eye and acquisition of the ocular characteristic by the ocular characteristic acquiring unit based on a determination result by the determining unit.

## Description

### {Technical Field}

The presently disclosed subject matter relates to an ophthalmic device that measures an ocular characteristic of a subject eye and a control method thereof.

### {Background Art}

In ophthalmology, various ocular characteristics such as an eye refractivity, an intraocular pressure, a number of corneal endothelial cells of a subject eye are acquired (e.g., measured, image-captured, observed) by an ophthalmic device. In this case, from the viewpoint such as precision, likelihood and image quality of an ocular characteristic to be acquired, positioning, that is, alignment of a measurement head (ocular characteristic acquiring unit) of the ophthalmic device with respect to a subject eye is extremely important. Accordingly, an ophthalmic device is well known which detects a relative position of a subject eye with respect to a measurement head and performs so-called full automatic alignment (hereinafter, simply called "auto alignment") that automatically aligns the measurement head with respect to the subject eye based on the detection result of the relative position.

However, even an ophthalmic device having such an auto alignment function may require time for acquiring a proper alignment state of the measurement head with respect to a subject eye, e.g., the subject eye having nystagmus or the subject eye of a restless child. Also, particularly when the eye refractivity is measured as an ocular characteristic of a subject eye, time is required for measuring the eye refractivity because it is difficult for a subject eye, e.g., the subject eye having nystagmus or the subject eye of a restless child to keep watching a fixation target for a certain period of time.

An ophthalmic device according to PTL 1 has a normal measurement mode in which a normal eye refractivity is measured and a continuous measurement mode in which the eye refractivity is measured continuously, as operation modes for measuring the eye refractivity of a subject eye. The continuous measurement mode is a mode in which the eye refractivity can be measured quickly by omitting a publicly known provisional measurement (preliminary measurement) and automatic fogging performed in the normal measurement mode.

The ophthalmic device according to PTL 1 switches its operation mode to a continuous measurement mode to measure the eye refractivity of a subject eye in response to a mode switching operation by a tester or when an eye refractivity measurement fails in the normal measurement mode. Thus, even when the subject eye has nystagmus or the subject is a child, a refractivity measurement can be performed continuously until a necessary measurement result of the eye refractivity is obtained. As a result, proper eye refractivity measurement according to the subject can be performed.

### {Citation List}

### {Patent Literature}

{PTL 1} Japanese Patent Application Laid-Open No. 2019-208590

### {Summary of Invention}

### {Technical Problem}

The ophthalmic device according to PTL 1 requires a tester to switch the operation mode of the ophthalmic device to the continuous measurement mode when the subject eye has nystagmus or a subject is a child, which imposes a problem of taking time and labor. Also, the ophthalmic device according to PTL 1 switches the operation mode to the continuous measurement mode when the eye refractivity measurement fails in the normal measurement mode, but, in this case, it wastes the eye refractivity measurement in the normal measurement mode, which imposes a problem of requiring additional time.

The presently disclosed subject matter was made in view of these circumstances, and it is an object of the invention to provide an ophthalmic device and a control method thereof which can acquire an ocular characteristic simply and in a short period of time even when the subject eye has nystagmus or the subject is a child.

### {Solution to Problem}

An ophthalmic device for achieving the object of the presently disclosed subject matter includes an ocular characteristic acquiring unit configured to acquire an ocular characteristic of a subject eye, a relatively-moving unit configured to relatively move the ocular characteristic acquiring unit relative to the subject eye, a detecting unit configured to detect a relative position of the subject eye relative to the ocular characteristic acquiring unit, a provisional alignment controlling unit configured to drive the relatively-moving unit based on a detection result by the detecting unit and perform provisional alignment of the ocular characteristic acquiring unit relative to the subject eye, a re-detecting unit configured to repeatedly detect the relative position by the detecting unit after completion of the provisional alignment, a determining unit configured to determine whether the ocular characteristic acquiring unit has been brought closer to the subject eye by a distance greater than or equal to a predetermined threshold value by the provisional alignment based on the detection result by the detecting unit before and after the provisional alignment; an operation mode selecting unit configured to select either one of a normal mode and a quick mode that is simpler than the normal mode as an operation mode for alignment of the ocular characteristic acquiring unit relative to the subject eye and acquisition of the ocular characteristic by the ocular characteristic acquiring unit based on a determination result by the determining unit, an alignment controlling unit configured to drive the relatively-moving unit based on the detection result by the detecting unit after completion of the provisional alignment and perform the alignment in the operation mode selected by the operation mode selecting unit, and a measurement controlling unit configured to cause acquisition of the ocular characteristic to be performed by the ocular characteristic acquiring unit in the operation mode selected by the operation mode selecting unit after completion of the alignment, wherein the operation mode selecting unit performs first selection processing that selects the normal mode as the operation mode for both of the alignment and the acquisition of the ocular characteristic when the determining unit determines that the ocular characteristic acquiring unit has been brought closer to the subject eye by a distance greater than or equal to the threshold value and selects the quick mode as the operation mode for at least one of the alignment and the acquisition of the ocular characteristic when the determining unit determines that the ocular characteristic acquiring unit has not been brought closer to the subject eye by a distance greater than or equal to the threshold value.

According to this ophthalmic device, an operation mode for alignment and acquisition of an ocular characteristic suitable for a subject eye (subject) can be automatically selected.

In the ophthalmic device according to the presently disclosed subject matter, the provisional alignment controlling unit can perform the provisional alignment based on the detection result by the detecting unit after completion of the provisional alignment, the ophthalmic device includes a repetition controlling unit configured to perform repetition control that causes the provisional alignment controlling unit, the re-detecting unit, and the determining unit to repeatedly operate one or more times after a first determination by the determining unit, and when the repetition control is performed, the operation mode selecting unit performs, instead of the first selection processing, second selection processing that selects the quick mode as the operation mode for at least one of the alignment and the acquisition of the ocular characteristic when the number of determinations that the determining unit determines that the ocular characteristic acquiring unit has not been brought closer to the subject eye by a distance greater than or equal to the threshold value has reached a predetermined number of times that is determined in advance, and selects the normal mode as the operation mode for both the alignment and the acquisition of the ocular characteristic when the number of determinations has not reached the predetermined number of times and the number of repetition controls has reached a predetermined upper limit number of times. Thus, an operation mode for alignment and acquisition of an ocular characteristic more suitable for a subject eye (subject) can be automatically selected.

The ophthalmic device for achieving the object of the presently disclosed subject matter includes an ocular characteristic acquiring unit configured to acquire an ocular characteristic of a subject eye, a relatively-moving unit configured to relatively move the ocular characteristic acquiring unit relative to the subject eye, a detecting unit configured to detect a relative position of the subject eye relative to the ocular characteristic acquiring unit, a first determining unit configured to determine whether the position of the ocular characteristic acquiring unit is within an allowable range of an alignment position of the ocular characteristic acquiring unit relative to the subject eye based on a detection result of the detecting unit; a second determining unit configured to, when the first determining unit determines a negative result, determine whether or not a shift flag is satisfied for shifting the operation mode for at least one of alignment of the ocular characteristic acquiring unit relative to the subject eye by the relatively-moving unit and acquisition of the ocular characteristic by the ocular characteristic acquiring unit from a normal mode to a quick mode that is simpler than the normal mode, an alignment controlling unit configured to, when the second determining unit determines a negative result, drive the relatively-moving unit and perform the alignment in the normal mode based on the detection result by the detecting unit, a repetition controlling unit configured to cause the detecting unit, the first determining unit, the second determining unit, and the alignment controlling unit to repeatedly operate until the first determining unit determines that the position is within the allowable range or until the second determining unit determines that the shift flag is satisfied; a normal-mode controlling unit configured to, when the first determining unit determines that the position is within the allowable range, cause the ocular characteristic acquiring unit to perform acquisition of the ocular characteristic in the normal mode, and a quick-mode controlling unit configured to, when the second determining unit determines that the shift flag is satisfied, cause at least one of the alignment by the alignment controlling unit and the acquisition of the ocular characteristic by the ocular characteristic acquiring unit to be performed in the quick mode.

According to this ophthalmic device, alignment and acquisition of an ocular characteristic can be automatically performed in an operation mode suitable for a subject eye (subject).

In the ophthalmic device according to another aspect of the presently disclosed subject matter, the second determining unit counts the number of times of the alignment by the alignment controlling unit and, based on whether or not the number of times of the alignment has reached a predetermined number of times that is determined in advance, determines the presence of the satisfaction of the shift flag.

In the ophthalmic device according to another aspect of the presently disclosed subject matter, the second determining unit performs processing of detecting a distance from the ocular characteristic acquiring unit to the subject eye every time the detecting unit detects the relative position and processing of counting the number of times that the distance exceeds the predetermined threshold value and determines the presence of the satisfaction of the shift flag based on whether or not the number of times the distance exceeds the threshold value has reached a predetermined number of times that is determined in advance.

In the ophthalmic device according to another aspect of the presently disclosed subject matter, the second determining unit calculates a variation across a plurality of detection results of the relative position over a plurality of detection results every time the detecting unit detects the relative position and determines the presence of the satisfaction of the shift flag based on whether the variation is higher than a predetermined threshold value or not.

A control method of an ophthalmic device for achieving the object of the presently disclosed subject matter includes a detecting step of detecting a relative position of a subject eye relative to an ocular characteristic acquiring unit configured to acquire an ocular characteristic of the subject eye, a provisional alignment step of relatively moving the ocular characteristic acquiring unit relative to the subject eye based on a detection result by the detecting step and performing provisional alignment of the ocular characteristic acquiring unit relative to the subject eye, a re-detecting step of repeatedly performing the detecting step after completion of the provisional alignment, a determining step of performing determination on whether the ocular characteristic acquiring unit has been brought closer to the subject eye by a distance greater than or equal to a predetermined threshold value by the provisional alignment based on the detection result by the detecting step before and after the provisional alignment, an operation mode selecting step of selecting either one of a normal mode and a quick mode that is simpler than the normal mode as an operation mode for alignment of the ocular characteristic acquiring unit relative to the subject eye and acquisition of the ocular characteristic by the ocular characteristic acquiring unit based on a determination result by the determining step, an alignment step of relatively moving the ocular characteristic acquiring unit relative to the subject eye based on the detection result by the detecting step after completion of the provisional alignment and performing the alignment in the operation mode selected by the operation mode selecting step, and an ocular characteristic acquiring step of causing acquisition of the ocular characteristic to be performed by the ocular characteristic acquiring unit in the operation mode selected by the operation mode selecting step after completion of the alignment, wherein the operation mode selecting step performs first selection processing that selects the normal mode as the operation mode for both of the alignment and the acquisition of the ocular characteristic when the determining step determines that the ocular characteristic acquiring unit has been brought closer to the subject eye by a distance greater than or equal to the threshold value and selects the quick mode as the operation mode for at least one of the alignment and the acquisition of the ocular characteristic when the determining step determines that the ocular characteristic acquiring unit has not been brought closer to the subject eye by a distance greater than or equal to the threshold value.

A control method of an ophthalmic device for achieving the object of the presently disclosed subject matter includes a detecting step of detecting a relative position of a subject eye relative to the ocular characteristic acquiring unit configured to acquire an ocular characteristic of the subject eye, a first determining step of determining whether the relative position detected by the detecting step is within an allowable range of an alignment position of the ocular characteristic acquiring unit relative to the subject eye, a second determining step of, when the first determining step determines a negative result, determining whether a shift flag is satisfied for shifting the operation mode for at least one of alignment of the ocular characteristic acquiring unit relative to the subject eye and acquisition of the ocular characteristic by the ocular characteristic acquiring unit from a normal mode to a quick mode that is simpler than the normal mode, an alignment step of relatively moving the ocular characteristic acquiring unit relative to the subject eye based on a detection result by the detecting step when the second determining step determines a negative result and performing the alignment in the normal mode, and a repeating step of repeatedly performing the detecting step, the first determining step, the second determining step, and the alignment step until the first determining step determines that the position is within the allowable range or until the second determining step determines that the shift flag is satisfied; a normal-mode controlling step of, when the first determining step determines that the position is within the allowable range, causing the ocular characteristic acquiring unit to perform acquisition of the ocular characteristic in the normal mode, and a quick-mode controlling step of, when the second determining step determines that the shift flag is satisfied, causing at least one of the alignment and the acquisition of the ocular characteristic by the ocular characteristic acquiring unit to be performed in the quick mode.

### {Advantageous Effects of Invention}

The presently disclosed subject matter can acquire an ocular characteristic simply and in a short period of time even when the subject eye has nystagmus or the subject is a child.

### {Brief Description of Drawings}

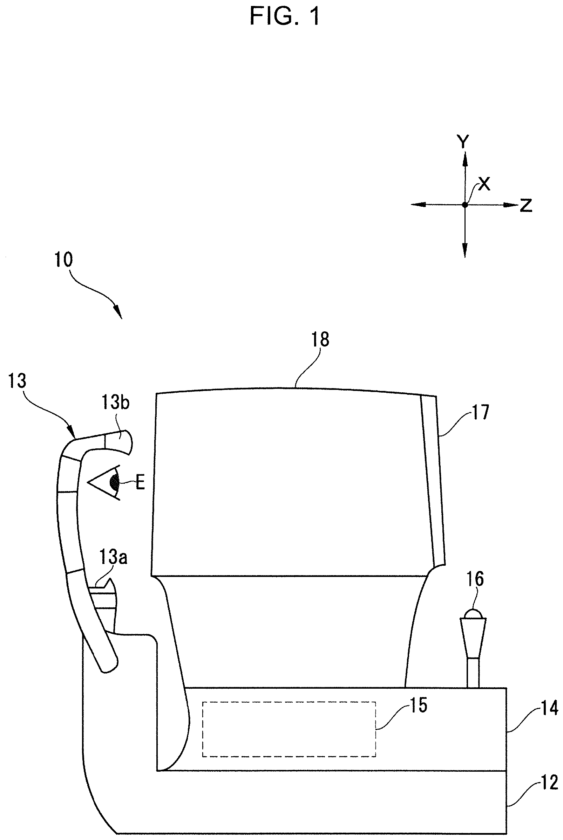

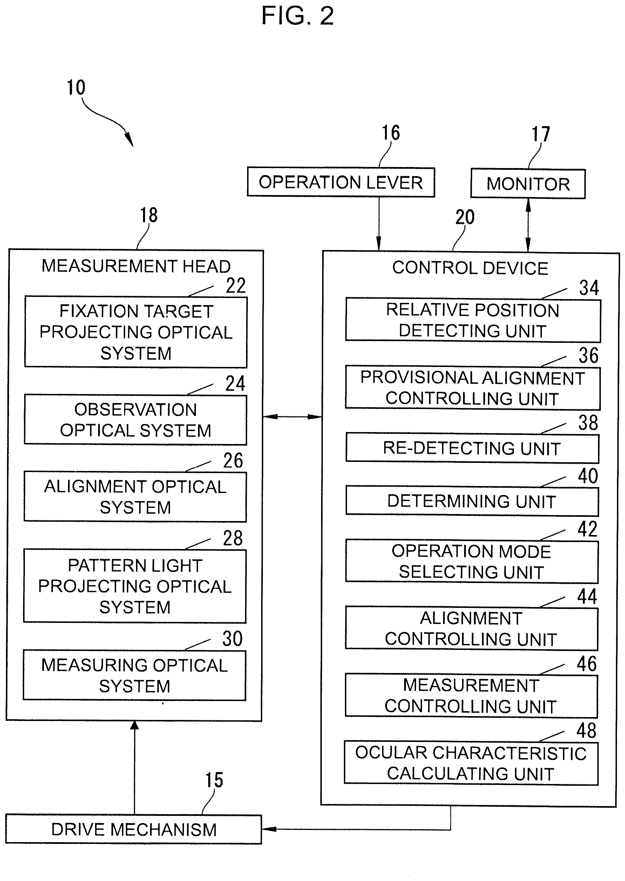

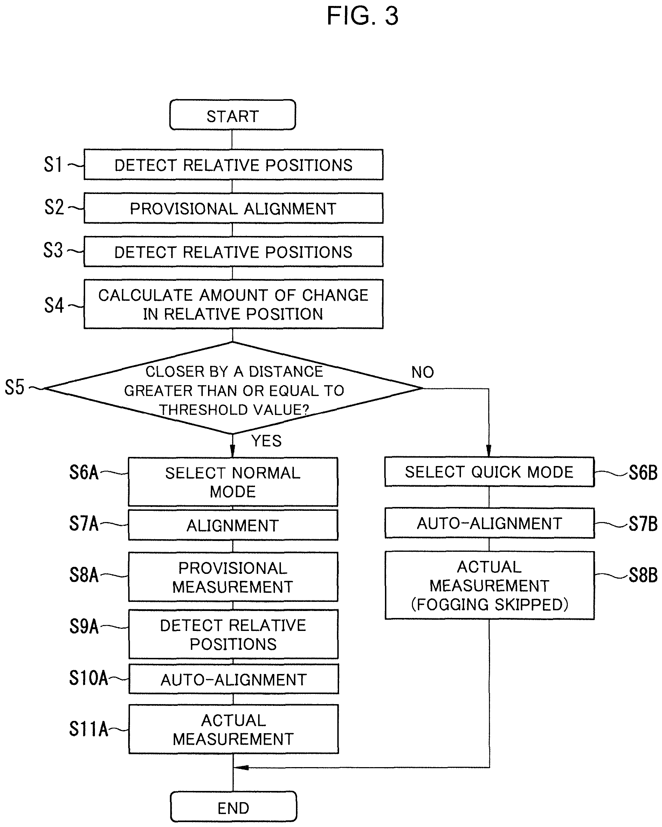

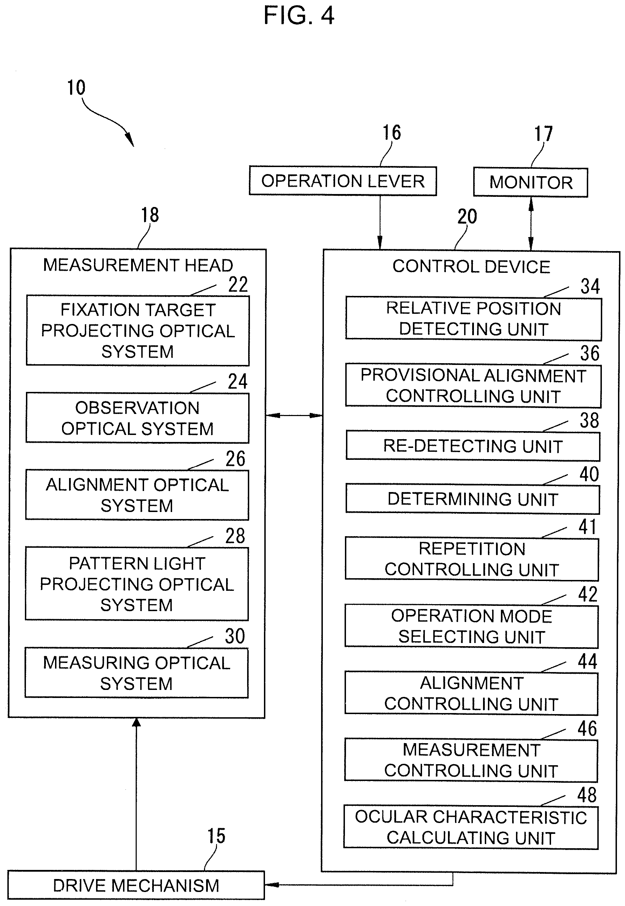

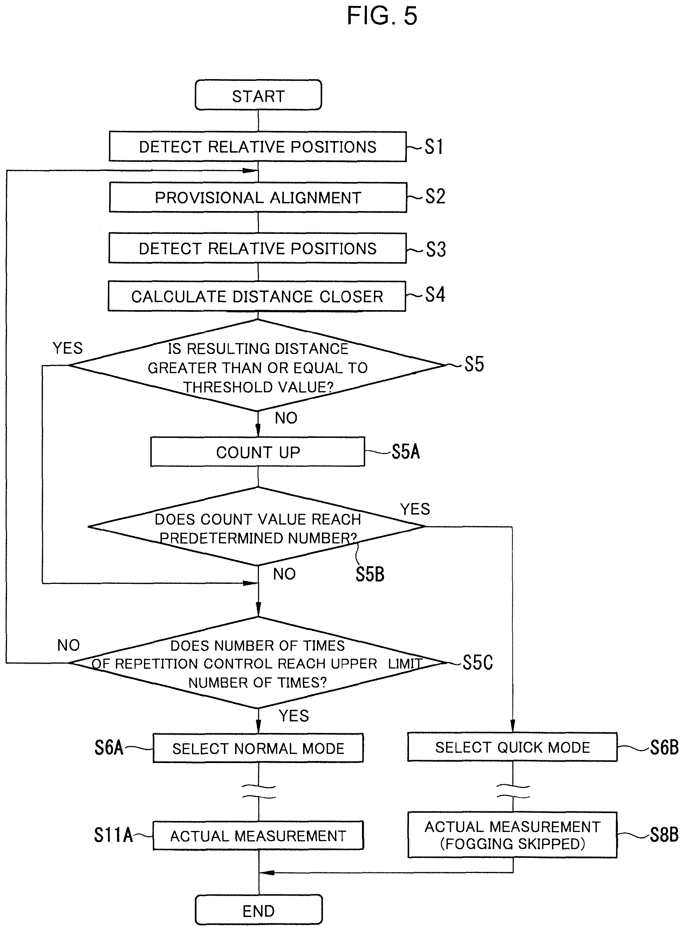

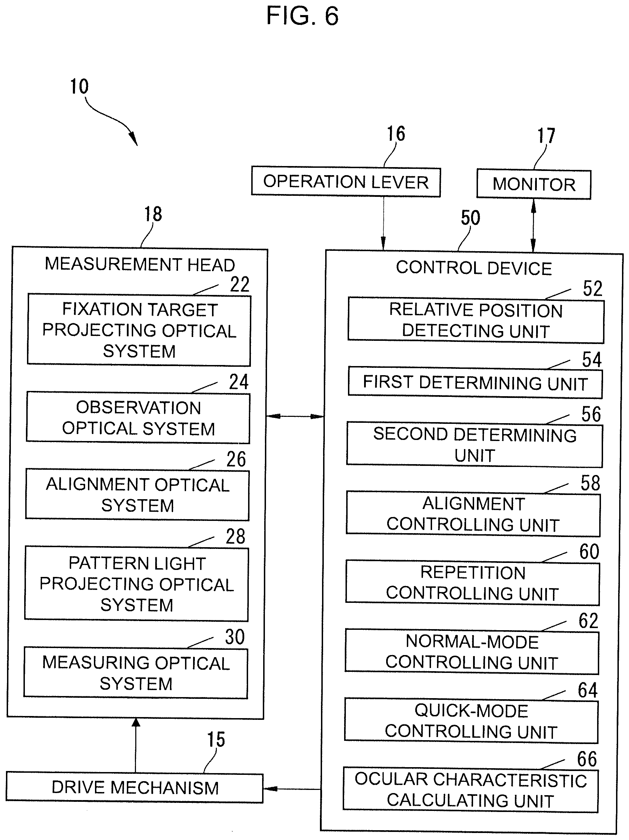

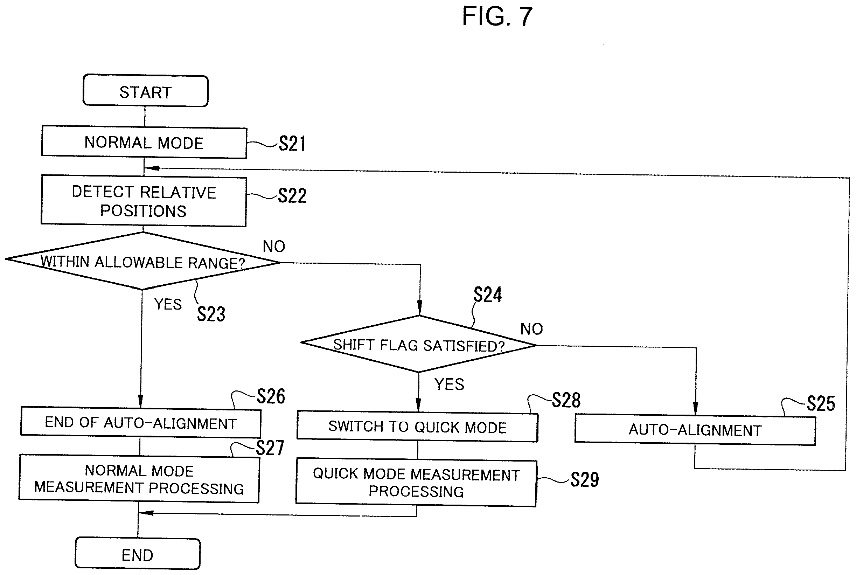

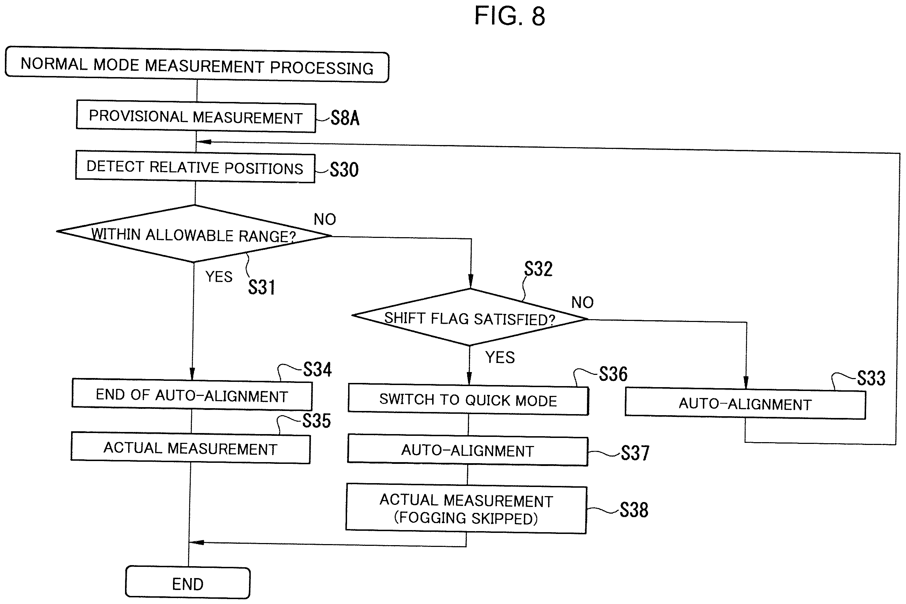

{Figure 1 }Figure 1 is a side view of an ophthalmic device according to a first embodiment.
{Figure 2}Figure 2 is a block diagram illustrating a schematic configuration of a measurement head and a control device according to the first embodiment.
{Figure 3}Figure 3 is a flowchart illustrating a flow of reflex measurement processing on a subject eye by the ophthalmic device according to the first embodiment.
{Figure 4}Figure 4 is a block diagram illustrating a schematic configuration of an ophthalmic device according to a second embodiment.
{Figure 5}Figure 5 is a flowchart illustrating a flow of reflex measurement processing on a subject eye by the ophthalmic device according to the second embodiment.
{Figure 6}Figure 6 is a block diagram illustrating a schematic configuration of an ophthalmic device according to a third embodiment.
{Figure 7}Figure 7 is a flowchart illustrating a flow of reflex measurement processing on a subject eye by the ophthalmic device according to the third embodiment.
{Figure 8} Figure 8 is a flowchart illustrating a flow of normal mode measurement processing in an ophthalmic device 10 according to a fourth embodiment.

### {Description of Embodiments}

### {First Embodiment}

Figure 1 is a side view of an ophthalmic device 10 according to a first embodiment. As illustrated in Figure 1, the ophthalmic device 10 may include an auto refractometer or an auto ref/keratometer, and the ophthalmic device 10 performs measurement of at least an eye refractivity as an ocular characteristic of a subject eye E (hereinafter, called reflex measurement). This ophthalmic device 10 includes a base 12, a face receiving unit 13, a frame 14, a drive mechanism 15, and a measurement head 18.

Here, the X direction in figures is the right-left direction with respect to a subject (interpupillary distance direction of the subject eye E), and the Y direction is a top-bottom direction, and the Z direction is a front-back direction (which is also called "working distance direction", parallel to a front direction close to a subject (subject eye E) and a back direction away from the subject).

The face receiving unit 13 is provided integrally to the base 12 at a position on the front side in the Z direction of the measurement head 18. This face receiving unit 13 has a jaw receiver 13a and a forehead protector 13b that are positionally adjustable in the Y direction and supports the face of the subject.

The frame 14 is provided on the base 12 and is movable in the XZ direction (front and back and right and left directions) with respect to the base 12. On this frame 14, the measurement head 18 and an operation lever 16 are provided.

The drive mechanism 15 corresponds to the relatively-moving unit of the presently disclosed subject matter and movably holds the measurement head 18 in the XYZ directions. The drive mechanism 15 is configured by a publicly known actuator, not illustrated, that moves the measurement head 18 in the XYZ directions and moves the measurement head 18 in each direction of the XYZ directions. Thus, driving the drive mechanism 15 under control of the control device 20, which is described later, enables auto-alignment in the XYZ directions of the measurement head 18 relative to the subject eye E. Also, driving the drive mechanism 15 in accordance with an operation with the operation lever 16, which is described later, enables manual alignment in the XYZ directions of the measurement head 18 relative to the subject eye E.

The operation lever 16 is an operation member that is provided on the frame 14 and is operated to move the measurement head 18 in each direction of XYZ axes. For example, when the operation lever 16 is operated to tilt in the Z direction (front-back direction) or X direction (right-left direction), the measurement head 18 is moved in the Z direction or the X direction by the drive mechanism 15. Also, when the operation lever 16 is operated to rotate around the longitudinal axis, the measurement head 18 is moved in the Y direction (top-bottom direction) by the drive mechanism 15 in accordance with the direction of the rotating operation. A measurement button is provided at the top of the operation lever 16, for starting a reflex measurement on the subject eye E by the ophthalmic device 10.

The measurement head 18 corresponds to the ocular characteristic acquiring unit of the presently disclosed subject matter and has a reflex measurement function for the subject eye E. Within this measurement head 18, various optical systems corresponding to the reflex measurement are provided.

A monitor 17 is, for example, a touch-panel type liquid crystal display device and is provided on a surface on the back side in the Z direction of the measurement head 18. This monitor 17 may display an observation image of an anterior eye part of the subject eye E to be used for alignment of the measurement head 18, a measurement result of an eye refractivity of the subject eye E obtained by the measurement head 18, and an input screen for performing operations (setting) relating to a reflex measurement, for example.

Figure 2 is a block diagram illustrating a schematic configuration of the measurement head 18 and the control device 20 according to the first embodiment. As illustrated in Figure 2, the measurement head 18 includes a fixation target projecting optical system 22, an observation optical system 24, an alignment optical system 26, a pattern light projecting optical system 28, and a measuring optical system 30. Since detail configurations of each of the optical systems are publicly known technologies, specific description is omitted here.

The fixation target projecting optical system 22 projects target light of a fixation target to the fundus of the subject eye E in order to achieve a fixation or fogging state of the subject eye E. The observation optical system 24 is an anterior eye camera that observes an anterior eye part of the subject eye E and outputs an observation image (image data) obtained by capturing an image of the anterior eye part to the control device 20. Thus, the observation image of the anterior eye part is displayed on the monitor 17 by the control device 20.

The alignment optical system 26 is provided to detect a relative position of the measurement head 18 relative to the subject eye E. This alignment optical system 26 projects various kinds of alignment index light (such as a bright point image) toward the subject eye E. Thus, an image of return light of the alignment index light reflected at the cornea of the subject eye E is captured by the observation optical system 24. Then, based on the captured image (image data) of the return light obtained by the observation optical system 24, manual alignment by a tester or auto-alignment by the control device 20 is performed. According to this embodiment (the same is true for the second embodiment, which is described later), auto-alignment is performed as the alignment.

The pattern light projecting optical system 28 projects a ring-shaped pattern light to the fundus of the subject eye E. Thus, the fundus reflected light of the pattern light projected to the fundus of the subject eye E is received by the measuring optical system 30. The shape of this fundus reflected light is distorted due to the eye refractivity of the subject eye E.

The measuring optical system 30 captures an image of (or receives) the fundus reflected light of the pattern light from the fundus of the subject eye E with an image pick-up device to obtain a captured image (image data) of the fundus reflected light. This captured image includes a ring image. Then, the measuring optical system 30 outputs the captured image of the fundus reflected light to the control device 20.

The control device 20 includes an arithmetic circuit including various kinds of processors and a memory. The various kinds of processors include a central processing unit (CPU), a graphics processing unit (GPU), a digital signal processing unit (DSP), an application specific integrated circuit (ASIC) or a programmable logic device (such as simple programmable logic devices (SPLD), complex programmable logic device (CPLD), and field programmable gate arrays (FPGA)). Various kinds of functions of the control device 20 may be implemented by one processor or may be implemented by processors of the same kind or of different kinds.

The drive mechanism 15, the operation lever 16, the monitor 17, and each optical system within the measurement head 18 are connected to the control device 20. This control device 20 controls the drive mechanism 15 and each optical system within the measurement head 18 so as to perform auto-alignment and reflex measurement.

Also, before the aforementioned auto-alignment and reflex measurement are performed, the control device 20 automatically selects an operation mode for the auto-alignment and reflex measurement suitable for the subject eye E (subject) and performs auto-alignment and reflex measurement in accordance with the selected operation mode. These operation modes include a normal mode and a quick mode.

The quick mode is a mode in which auto-alignment and reflex measurement can be performed more simply than the normal mode and is suitable for reflex measurement on the subject eye E, for example, the subject eye having nystagmus or the subject eye of a restless child. The term "performed simply" herein refers to performing alignment and reflex measurement with, for example, reduced accuracy but at a higher speed (in a short period of time). An example of reflex measurement and auto-alignment for each operation mode (normal mode, quick mode) are described below.

The reflex measurement in the normal mode includes, for example, provisional measurement (which may also be called preliminary measurement or rough measurement) and an actual measurement. The provisional measurement is performed for the purpose of properly producing fogging of the subject eye E in the actual measurement and measures an eye refractivity of the subject eye E with a fixation target presented to the subject eye E. The actual measurement produces fogging of the subject eye F based on the eye refractivity of the subject eye E obtained in the provisional measurement and measures the eye refractivity of the subject eye E having that state.

The reflex measurement in the quick mode only performs, for example, the actual measurement by omitting (skipping) the provisional measurement. The actual measurement in this quick mode is different from the actual measurement in the normal mode and measures the eye refractivity of the subject eye E without producing fogging of the subject eye E (fogging skipped).

The auto-alignment in the normal mode is performed before each of the provisional measurement and the actual measurement. The auto-alignment in the quick mode is performed before the actual measurement. Also, for the auto-alignment in the quick mode, the allowable range (target value) of the alignment position of the measurement head 18 relative to the subject eye E after completion of the alignment is set wider than that of the normal mode.

Performing the auto-alignment in the normal mode before the actual measurement (re-alignment) may be omitted. In this case, the normal mode and the quick mode for the auto-alignment are distinguished in accordance with the width of the aforementioned allowable range.

The control device 20 executes a control program stored in a storage device, not illustrated, to function as a relative position detecting unit 34, a provisional alignment controlling unit 36, a re-detecting unit 38, a determining unit 40, an operation mode selecting unit 42, an alignment controlling unit 44, a measurement controlling unit 46, and an ocular characteristic calculating unit 48. Here, a term "- unit" in the description of the control device 20 may refer to "- circuit", "- device", or "- apparatus". In other words, each of those described as "- unit" may be configured by any of firmware, software, and hardware or a combination thereof.

The relative position detecting unit 34 configures the detecting unit of the preset invention along with the alignment optical system 26 and the observation optical system 24. When a start operation for a reflex measurement is performed by a tester, the relative position detecting unit 34 controls the alignment optical system 26 and the observation optical system 24 so as to perform relative position detecting processing that detects a relative position of the subject eye E relative to the measurement head 18. More specifically, the relative position detecting unit 34 sequentially performs projection of alignment index light to the cornea of the subject eye E by the alignment optical system 26, capturing of an image of return light of the alignment index light from the cornea by the observation optical system 24, and output of the captured image of the return light from the observation optical system 24 to the relative position detecting unit 34. Then, the relative position detecting unit 34 detects the relative position of the subject eye E relative to the measurement head 18 by a publicly known method based on the captured image input from the observation optical system 24.

The provisional alignment controlling unit 36 drives the drive mechanism 15 to relatively move the measurement head 18 relative to the subject eye E so as to perform provisional alignment of the measurement head 18 relative to the subject eye E based on a detection result of the relative position of the subject eye E by the relative position detecting unit 34 (hereinafter, simply called "position detection result by the relative position detecting unit 34" for short). This provisional alignment is not particularly limited when it is movement that brings the measurement head 18 relatively close to the subject eye E. For example, like normal alignment (actual alignment), the measurement head 18 is relatively moved into the allowable range of the alignment position of the measurement head 18 relative to the subject eye E or the measurement head 18 is relatively moved to the middle of the path from the current position into the aforementioned allowable range.

The re-detecting unit 38 controls the relative position detecting unit 34 so as to repeatedly perform the relative position detection processing described above after completion of the provisional alignment by the provisional alignment controlling unit 36. Thus, by the relative position detecting unit 34, the relative position of the subject eye E relative to the measurement head 18 after completion of the provisional alignment is detected. Also, when the normal mode is selected by the operation mode selecting unit 42, which is described in detail later, the re-detecting unit 38 of this embodiment controls the relative position detecting unit 34 so as to repeatedly perform the relative position detection processing described above, also after provisional measurement of a reflex measurement and before an actual measurement.

The determining unit 40 acquires a position detection result by the relative position detecting unit 34 upon start operation of a reflex measurement (before the provisional alignment) and a position detection result by the relative position detecting unit 34 after completion of the provisional alignment, that is, position detection results by the relative position detecting unit 34 before and after the provisional alignment. Then, based on these position detection results, the determining unit 40 calculates an amount of change between the relative positions of the subject eye E before and after the provisional alignment (hereinafter, called "amount of change in relative position"). This amount of change in relative position may be calculated for each of individual XYZ directions or may be calculated by a square root of sum of squares {R = √(X² + Y² + Z²)}. Then, based on the calculation result of the amount of change in relative position, the determining unit 40 determines whether or not the measurement head 18 has been brought closer to the subject eye E by the provisional alignment by a distance greater than or equal to a predetermined threshold value and outputs the determination result to the operation mode selecting unit 42.

Here, the threshold value for the distance is determined based on the head moving distance that is a distance that the measurement head 18 is actually moved by the provisional alignment. In this embodiment, for example, in consideration of an error in the provisional alignment and a detection error of the relative position detecting unit 34, a value acquired by subtracting those errors from the head moving distance is defined as a threshold value.

Based on the determination result by the determining unit 40, the operation mode selecting unit 42 performs selection processing (corresponding to the first selection processing of the presently disclosed subject matter) of selecting either normal mode or quick mode as the operation mode for auto-alignment and reflex measurement. Here, when the determining unit 40 determines that the measurement head 18 has been brought closer to the subject eye E by the provisional alignment by a distance greater than or equal the predetermined threshold value, it is assumed that no movement occurs in the subject eye E while the measurement head 18 is moving by the head moving distance described above by the provisional alignment. Thus, the operation mode selecting unit 42 selects the normal mode as the operation mode for auto-alignment and reflex measurement.

On the other hand, when the determining unit 40 determines a negative result, it is assumed that a movement is occurring in the subject eye E, for example, the subject eye E has nystagmus or the subject is a restless child while the measurement head 18 is moving by the head moving distance described above by the provisional alignment. Thus, the operation mode selecting unit 42 selects the quick mode as the operation mode for auto-alignment and reflex measurement.

According to this embodiment, when the determining unit 40 determines a negative result, that is, when the determining unit 40 determines that the measurement head 18 is not brought closer to the subject eye E by the provisional alignment by a distance greater than or equal the predetermined threshold value, the operation mode selecting unit 42 selects the quick mode, but the presently disclosed subject matter is not limited thereto. For example, when the measurement head 18 has been brought closer to the subject eye E by the provisional alignment over a certain amount than the head moving distance described above, it is also assumed that a movement is occurring in the subject eye E during the provisional alignment.

Accordingly, based on the detection results of the relative positions of the subject eye E before and after the provisional alignment, the determining unit 40 may determine whether or not the measurement head 18 has been brought closer to the subject eye E by the provisional alignment by a distance within a predetermined threshold value range (within the distance range). In this case, when the determining unit 40 determines that the measurement head 18 has been brought closer by a distance within the threshold value range described above, the operation mode selecting unit 42 selects the normal mode, or, when the determining unit 40 determines a negative result, selects the quick mode.

In accordance with the operation mode selected by the operation mode selecting unit 42, the alignment controlling unit 44 drives the drive mechanism 15 to relatively move the measurement head 18 relative to the subject eye E so that auto-alignment of the measurement head 18 relative to the subject eye E is performed. When the operation mode selecting unit 42 selects the normal mode, the alignment controlling unit 44 performs a (first) auto-alignment preceding a provisional measurement for a reflex measurement based on the position detection result by the relative position detecting unit 34 after completion of the provisional alignment. Also, after a provisional measurement for a reflex measurement and before an actual measurement, the alignment controlling unit 44 performs a (second) auto-alignment preceding the actual measurement for reflex measurement based on a new position detection result by the relative position detecting unit 34.

On the other hand, when the operation mode selecting unit 42 selects the quick mode, the alignment controlling unit 44 performs auto-alignment preceding an actual measurement for reflex measurement based on a relative position detection result of the subject eye E by the relative position detecting unit 34 after completion of the provisional alignment. Also, at this point of time, the alignment controlling unit 44 sets an allowable range (target value) of the alignment position of the measurement head 18 relative to the subject eye E after completion of the auto-alignment wider than that of the normal mode. Thus, auto-alignment in the quick mode can be performed more simply (with low precision but at a high speed) than auto-alignment in the normal mode.

After completion of the auto-alignment, in accordance with the operation mode selected by the operation mode selecting unit 42, the measurement controlling unit 46 controls the fixation target projecting optical system 22, the pattern light projecting optical system 28, and the measuring optical system 30 so as to perform reflex measurement on the subject eye E. When the operation mode selecting unit 42 selects the normal mode, the measurement controlling unit 46 starts a provisional measurement for the reflex measurement on the subject eye E after completion of the first auto-alignment. More specifically, the measurement controlling unit 46 performs presenting a fixation target to the subject eye E by the fixation target projecting optical system 22, projecting pattern light to the fundus of the subject eye E by the pattern light projecting optical system 28, capturing an image of fundus reflected light of the pattern light by the measuring optical system 30, and outputting the captured image of the fundus reflected light from the measuring optical system 30 to the ocular characteristic calculating unit 48. Thus, a provisional measurement value (also called "preliminary measurement value" or "rough measurement value") for the eye refractivity of the subject eye E is obtained in the ocular characteristic calculating unit 48, which is described later.

Then, after completion of the second auto-alignment, the measurement controlling unit 46 starts an actual measurement for reflex measurement on the subject eye E. More specifically, based on the provisional measurement value of the eye refractivity of the subject eye E obtained by the provisional measurement, the measurement controlling unit 46 controls the fixation target projecting optical system 22 so as to change the fixation target to a fogging state, producing fogging of the subject eye E. Then, the measurement controlling unit 46 performs projecting pattern light to the fundus of the subject eye E by the pattern light projecting optical system 28, capturing an image of fundus reflected light of the pattern light by the measuring optical system 30, and outputting the captured image of the fundus reflected light from the measuring optical system 30 to the ocular characteristic calculating unit 48. The reflex measurement (provisional measurement, actual measurement) in the normal mode is a publicly known technology and may be performed in various methods other than the aforementioned method.

On the other hand, when the operation mode selecting unit 42 selects the quick mode, the measurement controlling unit 46 starts an actual measurement by skipping the provisional measurement after completion of an auto-alignment. More specifically, the measurement controlling unit 46 skips producing fogging of the subject eye E by the fixation target projecting optical system 22 and, in this state, performs projecting pattern light to the fundus of the subject eye E by the pattern light projecting optical system 28, capturing an image of fundus reflected light of the pattern light by the measuring optical system 30, and outputting the captured image of the fundus reflected light from the measuring optical system 30 to the ocular characteristic calculating unit 48. Thus, reflex measurement in the quick mode can be performed more easily (with lower precision but at a higher speed) than reflex measurement in the normal mode.

Based on the captured image input from the measuring optical system 30 upon a reflex measurement on the subject eye E, the ocular characteristic calculating unit 48 calculates the eye refractivity (such as spherical diopter, astigmatism diopter or astigmatism axis angle) of the subject eye E by a publicly known method. The ocular characteristic calculating unit 48 calculates, in the normal mode, a provisional measurement value of the eye refractivity of the subject eye E based on the captured image obtained by the provisional measurement in the normal mode and calculates an actual measurement value of the eye refractivity of the subject eye E based on the captured image obtained by the subsequent actual measurement. Also, the ocular characteristic calculating unit 48 calculates an actual measurement value of the eye refractivity of the subject eye E in the quick mode based on the captured image obtained by the actual measurement.

### {Effects of First Embodiment}

Figure 3 is a flowchart illustrating a flow of reflex measurement processing on the subject eye E by the ophthalmic device 10 of the first embodiment according to the control method for the ophthalmic device of the presently disclosed subject matter. As illustrated in Figure 3, when a tester performs a start operation for a reflex measurement, the relative position detecting unit 34 controls the alignment optical system 26 and the observation optical system 24 so as to perform the aforementioned relative position detecting processing and detects a relative position of the subject eye E relative to the measurement head 18 (step S 1, corresponding to the detecting step of the presently disclosed subject matter).

Then, based on the position detection result by the relative position detecting unit 34, the provisional alignment controlling unit 36 drives the drive mechanism 15 so as to perform a provisional alignment of the measurement head 18 relative to the subject eye E (step S2, corresponding to the provisional alignment step of the presently disclosed subject matter).

Then, when the provisional alignment is complete, the re-detecting unit 38 causes the relative position detecting unit 34 to repeatedly perform the relative position detection processing so that the relative position detecting unit 34 detects a relative position of the subject eye E relative to the measurement head 18 after completion of the provisional alignment (step S3, corresponding to the re-detecting step of the presently disclosed subject matter). Thus, the relative positions of the subject eye E before and after the provisional alignment are detected.

When the detection of the relative positions of the subject eye E after completion of the provisional alignment is complete, the determining unit 40 calculates an amount of change in relative position of the subject eye E between before and after the provisional alignment (step S4). Then, based on the calculation result of the amount of change in relative position of the subject eye E, the determining unit 40 determines whether or not the measurement head 18 has been brought closer to the subject eye E by the provisional alignment by a distance greater than or equal to a predetermined threshold value (step S5, the determining step of the presently disclosed subject matter). Thus, the presence of a movement of the subject eye E during the provisional alignment can be determined, that is, whether or not the subject eye E has nystagmus or the subject is a restless child can be determined. The determining unit 40 outputs the determination result to the operation mode selecting unit 42.

Then, when the determining unit 40 determines that the measurement head 18 has been brought closer to the subject eye E by the provisional alignment by a distance greater than or equal to a predetermined threshold value (YES in step S5), the operation mode selecting unit 42 selects the normal mode as an operation mode for auto-alignment and reflex measurement (step S6A). Also, conversely, when the determining unit 40 determines a negative result (NO in step S5), the operation mode selecting unit 42 selects the quick mode as the operation mode for auto-alignment and reflex measurement (step S6B). Here, step S6A and step S6B correspond to the operation mode selecting step of the presently disclosed subject matter. Thus, before the auto-alignment and reflex measurement, an operation mode for the auto-alignment and reflex measurement suitable for the subject eye E (subject) can be automatically selected.

When the operation mode selecting unit 42 selects the normal mode (step S6A), the alignment controlling unit 44 drives the drive mechanism 15 to perform auto-alignment preceding a provisional measurement based on the detection result of the relative position of the subject eye E after completion of the provisional alignment, which is obtained in step S3 (step S7A).

When the auto-alignment preceding a provisional measurement is complete, the measurement controlling unit 46 controls the fixation target projecting optical system 22, the pattern light projecting optical system 28, and the measuring optical system 30 so as to perform a provisional measurement for reflex measurement (step S8A). When this provisional measurement is complete, the ocular characteristic calculating unit 48 calculates a provisional measurement value of the eye refractivity of the subject eye E.

When the calculation of a provisional measurement value is complete, the re-detecting unit 38 causes the relative position detecting unit 34 to repeatedly perform the relative position detection processing so that the relative position detecting unit 34 re-detects a relative position of the subject eye E relative to the measurement head 18 before an actual measurement (step S9A). Then, based on this detection result of the relative position, the alignment controlling unit 44 drives the drive mechanism 15 so as to perform an auto-alignment (step S10A, corresponding to the alignment step of the presently disclosed subject matter).

When the auto-alignment preceding an actual measurement is complete, the measurement controlling unit 46 controls the fixation target projecting optical system 22, the pattern light projecting optical system 28, and the measuring optical system 30 again so as to perform an actual measurement for reflex measurement with the subject eye E having a fogging state (step S11A, corresponding to the ocular characteristic acquiring step of the presently disclosed subject matter). When this actual measurement is complete, the ocular characteristic calculating unit 48 calculates an actual measurement value of the eye refractivity of the subject eye E.

In this way, in the normal mode, normal reflex measurement (provisional measurement, actual measurement) is performed so that the eye refractivity of the subject eye E can be measured with high precision.

On the other hand, when the operation mode selecting unit 42 selects the quick mode (step S6B), the alignment controlling unit 44 drives the drive mechanism 15 to perform an auto-alignment in the quick mode based on the detection result of the relative position of the subject eye E after completion of the provisional alignment, which is obtained in step S3 (step S7B, corresponding to the alignment step of the presently disclosed subject matter). At this point of time, the alignment controlling unit 44 sets a wider allowable range of the alignment position of the measurement head 18 relative to the subject eye E than that of the normal mode so that the auto-alignment can be simplified (increasing the speed).

When the auto-alignment preceding an actual measurement is complete, the measurement controlling unit 46 controls the pattern light projecting optical system 28, and the measuring optical system 30 again so as to perform an actual measurement for reflex measurement without producing fogging of the subject eye E (fogging skipped) (step S8B, corresponding to the ocular characteristic acquiring step of the presently disclosed subject matter). When this actual measurement is complete, the ocular characteristic calculating unit 48 calculates an actual measurement value of the eye refractivity of the subject eye E.

In this way, in the quick mode, by increasing a range of the allowable value for auto-alignment, reducing the number of times of auto-alignment, skipping a provisional measurement, and/or further performing fogging skip in an actual measurement, the eye refractivity of the subject eye E can be measured more simply (with lower precision but more quickly) than the normal mode. Time is required for reflex measurement in the normal mode because it is difficult for the subject eye E having nystagmus or the subject eye of a restless child to keep watching a fixation target for a certain degree of time. Also, since a child may be more uncooperative toward measurement when the measurement time is longer, the measurement may possibly fail. On the other hand, in a reflex measurement in the quick mode, the measurement can be completed in a short period of time though the precision of the measurement of the eye refractivity may be lower by skipping a part of the processing.

In this way, according to this embodiment, a provisional alignment is performed before an auto-alignment and a reflex measurement, and, based on the relative positions of the subject eye E before and after the provisional measurement, the operation mode for the auto-alignment and reflex measurement suitable for the subject eye E (subject) can be automatically selected. Thus, this embodiment may eliminate the needs for manually switching the operation mode by the tester as disclosed in PTL 1 or for holding a measurement in the quick mode until measurement of the eye refractivity in the normal mode fails. As a result, a reflex measurement can be performed quickly in the quick mode for the subject eye E having nystagmus or the subject eye of a restless child, for example. Thus, even when the subject eye E has nystagmus or the subject is a child, a reflex measurement can be performed simply and in a short period of time.

### {Second Embodiment}

Figure 4 is a block diagram illustrating a schematic configuration of an ophthalmic device 10 according to a second embodiment. According to the ophthalmic device 10 of the first embodiment, based on the relative positions of the subject eye E before and after one provisional measurement, the operation mode for auto-alignment and reflex measurement is selected. On the other hand, in the ophthalmic device 10 of the second embodiment, instead of performing the selection of an operation mode based on the relative positions of the subject eye E before and after one provisional alignment, provisional alignments are performed several times, and an operation mode is selected based on relative positions of the subject eye E before and after each provisional alignment.

As illustrated in Figure 4, the ophthalmic device 10 of the second embodiment has basically the same configuration as that of the ophthalmic device 10 of the first embodiment except that a control device 20 functions as a repetition controlling unit 41. Thus, like references are given to like parts in terms of their function or configuration between the aforementioned first embodiment and the second embodiment, and repetitive description is omitted.

Although a provisional alignment controlling unit 36 of the second embodiment is basically the same as the provisional alignment controlling unit 36 of the first embodiment, the provisional alignment controlling unit 36 further has functionality to perform a provisional alignment based on a position detection result by the relative position detecting unit 34, which is detected after completion of the last provisional alignment.

The repetition controlling unit 41 performs repetition control that causes the provisional alignment controlling unit 36, a re-detecting unit 38, and a determining unit 40 to repeatedly operate one or more times after the first determination by the determining unit 40. That the upper limit number of times of the repetition control is predetermined. Thus, the provisional alignment, relative position detection of the subject eye E and determination by the determining unit 40 are performed several times.

An operation mode selecting unit 42 of the second embodiment counts the number of determinations that the determining unit 40 determines a negative result. Then, the operation mode selecting unit 42 selects the quick mode as the operation mode for auto-alignment and reflex measurement when the count value of the number of determinations reaches a predetermined number and selects the normal mode as the operation mode when the number of times of repetition control reaches the upper limit number of times without the count value reaching the predetermined number. This selection processing corresponds to the second selection processing of the presently disclosed subject matter.

Figure 5 is a flowchart illustrating a flow of reflex measurement processing on the subject eye E by the ophthalmic device 10 according to the second embodiment. As illustrated in Figure 5, after the processing from step S1 to step S5 described according to the first embodiment (see Figure 3) is performed and when the determining unit 40 determines a negative result (NO in step S5), the operation mode selecting unit 42 counts up the counter (the count value is incremented by +1) (step S5A).

Then, when the count value of the counter described above does not reach a predetermined number determined in advance (NO in step S5B) or when the determining unit 40 determines that the measurement head 18 has been brought closer to the subject eye E by a distance greater than or equal a threshold value (YES in step S5), the repetition controlling unit 41 starts repetition control (NO in step S5C). Thus, based on the detection result by the relative position detecting unit 34 in step S3, the provisional alignment controlling unit 36 drives the drive mechanism 15 so as to perform a provisional alignment (step S2). After completion of the provisional alignment, the processing from step S4 to step S5B is repeatedly performed.

Subsequently, when the count value of the counter (the number of times of determination that the determining unit 40 determines a negative result) does not reach the predetermined number (NO in step S5B) and when the number of times of the repetition control by the repetition controlling unit 41 does not reach the upper limit number of times (NO in step S5C), the repetition control (step S2 through step S5B) is repeatedly performed.

Then, when the count value of the counter reaches the predetermined number of times in step S5B (YES in step S5B), the operation mode selecting unit 42 selects the quick mode as the operation mode for auto-alignment and reflex measurement (step S6B). Also, when the number of times of the repetition control reaches the upper limit number of times in step S5C (YES in step S5C), the operation mode selecting unit 42 selects the normal mode as the operation mode for auto-alignment and reflex measurement (step S6A).

Since the processing in subsequent steps illustrated in Figure 3 is the same as the processing of the first embodiment, specific description is omitted here.

In this way, since, according to the second embodiment, the provisional alignment is performed several times and, based on the results of determinations by the determining unit 40, the operation mode for auto-alignment and reflex measurement is selected, selection of the operation mode more suitable for the subject eye E (subject) than the first embodiment described above can be performed.

### {Third Embodiment}

Figure 6 is a block diagram illustrating a schematic configuration of an ophthalmic device 10 according to a third embodiment. According to the ophthalmic device 10 of each of the aforementioned embodiments, based on the relative positions of the subject eye E before and after a provisional alignment, the operation mode for auto-alignment and reflex measurement is selected, and auto-alignment and reflex measurement are performed in accordance with the selected operation mode. On the other hand, the ophthalmic device 10 of the third embodiment performs auto-alignment and reflex measurement in an operation mode suitable for the subject eye E (subject) by a method different from each of the aforementioned embodiments.

As illustrated in Figure 6, the ophthalmic device 10 of the third embodiment has a configuration that is basically the same as that of the ophthalmic device 10 of each of the aforementioned embodiments except that a control device 50 different from that of each of the aforementioned embodiments is provided. Thus, like references are given to like parts in terms of their function or configuration between the aforementioned embodiments and the third embodiment, and repetitive description is omitted.

The control device 50 of the third embodiment executes a control program stored in a storage device, not illustrated, to function as a relative position detecting unit 52, a first determining unit 54, a second determining unit 56, an alignment controlling unit 58, a repetition controlling unit 60, a normal-mode controlling unit 62, a quick-mode controlling unit 64, and an ocular characteristic calculating unit 66. According to the third embodiment, as an initial setting, the operation mode for auto-alignment and reflex measurement is set as the normal mode.

The relative position detecting unit 52 is basically the same as the relative position detecting unit 34 according to each of the aforementioned embodiments and configures the detecting unit of the preset invention along with the alignment optical system 26 and the observation optical system 24. When a start operation for a reflex measurement is performed by a tester, the relative position detecting unit 52 performs relative position detecting processing described above to detect a relative position of the subject eye E relative to the measurement head 18.

Based on the position detection result by the relative position detecting unit 52, the first determining unit 54 determines whether or not the current position of the measurement head 18 is within an allowable range (target value) of the alignment position of the measurement head 18 relative to the subject eye E after completion of the auto-alignment.

When the first determining unit 54 determines a negative result, the second determining unit 56 operates and determines whether or not a shift flag for shifting the operation mode for auto-alignment and reflex measurement from the normal mode to the quick mode is satisfied. For example, the second determining unit 56 counts the number of times of the auto-alignment in the normal mode executed by the alignment controlling unit 58, which is described later and, based on whether or not the number of times of auto-alignment has reached a predetermined number of times that is determined in advance, and determines the presence of the satisfaction of the shift flag.

When the second determining unit 56 determines a negative result (the shift flag is not satisfied), the alignment controlling unit 58, based on the position detection result by the relative position detecting unit 34, drives the drive mechanism 15 so as to perform auto-alignment in the normal mode.

The repetition controlling unit 60 performs repetition control that causes the relative position detecting unit 52, the first determining unit 54, the second determining unit 56, and the alignment controlling unit 58 to repeatedly operate until the first determining unit 54 determines that the current position of the measurement head 18 is within the allowable range or until the second determining unit 56 determines that the shift flag is satisfied.

The normal-mode controlling unit 62 operates when the first determining unit 54 determines that the current position of the measurement head 18 is within the aforementioned allowable range. The normal-mode controlling unit 62 controls each optical system of the measurement head 18 and the alignment controlling unit 58 so as to perform normal mode measurement processing. This normal mode measurement processing includes, for example, a provisional measurement, detection of a relative position of the subject eye E, auto-alignment preceding an actual measurement, and an actual measurement, which are described in each of the aforementioned embodiments.

The quick-mode controlling unit 64 operates when the second determining unit 56 determines the satisfaction of the shift flag. The quick-mode controlling unit 64 controls each optical system of the measurement head 18 and the alignment controlling unit 58 so as to perform quick mode measurement processing. This quick mode measurement processing includes, for example, auto-alignment in the quick mode and an actual measurement (fogging skipped), which are described in each of the aforementioned embodiments.

The ocular characteristic calculating unit 66 is basically the same as the ocular characteristic calculating unit 48 of each of the aforementioned embodiments, and, in the normal mode, performs calculation of a provisional measurement value of an eye refractivity of the subject eye E in a provisional measurement and calculation of an actual measurement value of the eye refractivity of the subject eye E in an actual measurement and, in the quick mode, performs calculation of an actual measurement value of the eye refractivity of the subject eye E in an actual measurement.

Figure 7 is a flowchart illustrating a flow of reflex measurement processing on the subject eye E by the ophthalmic device 10 according to the third embodiment. As illustrated in Figure 7, the operation mode for auto-alignment and reflex measurement is set as the normal mode in advance (step S21).

When a tester executes a start operation for a reflex measurement, the relative position detecting unit 34 controls the alignment optical system 26 and the observation optical system 24 so as to perform the aforementioned relative position detecting processing, detecting a relative position of the subject eye E relative to the measurement head 18 (step S22, corresponding to the detecting step of the presently disclosed subject matter).

Then, the first determining unit 54 determines whether the current position of the measurement head 18 is within the aforementioned allowable range or not based on the position detection result by the relative position detecting unit 34 (step S23, corresponding to the first determining step of the presently disclosed subject matter). That the initial determination by the first determining unit 54 is executed in a state that no auto-alignment is performed. In this case, since the possibility that the current position of the measurement head 18 is within the allowable range is low, the possibility that the first determining unit 54 determines a negative result is high.

When the first determining unit 54 determines a negative result (NO in step S23), the second determining unit 56 determines whether the shift flag is satisfied or not (step S24, corresponding to the second determining step of the presently disclosed subject matter). The initial determination by the second determining unit 56 is also executed in a state that no auto-alignment is performed. Therefore, the number of times of auto-alignment does not reach a predetermined number of times, which is set in advance, and the second determining unit 56 determines a negative result (NO in step S24).

When the second determining unit 56 determines a negative result, based on the position detection result by the relative position detecting unit 34 in step S22, the alignment controlling unit 58 drives the drive mechanism 15 so as to perform an auto-alignment in the normal mode (step S25, corresponding to the alignment step of the presently disclosed subject matter).

Subsequently, until the first determining unit 54 determines that the current position of the measurement head 18 is within the aforementioned allowable range (YES in step S23) or when the second determining unit 56 determines the satisfaction of the shift flag (YES in step S24), the repetition controlling unit 60 executes the aforementioned repetition control (corresponding to the repetition controlling step of the presently disclosed subject matter). Thus, the processing from step S21 to step S25 is repeatedly performed one or more times.

When a problem does not occur such as the subject eye E having nystagmus or the subject eye of a restless child, performing one or more auto-alignments allows setting the current position of the measurement head 18 within the aforementioned allowable range. In this case, the first determining unit 54 determines that the current position of the measurement head 18 is within the allowable range (YES in step S23), and the aforementioned auto-alignment (repetition control) ends (step S26).

Then, the normal-mode controlling unit 62 controls each optical system of the measurement head 18, the alignment controlling unit 58, and the ocular characteristic calculating unit 66 so as to perform normal mode measurement processing (step S27, corresponding to the normal mode controlling step of the presently disclosed subject matter). Thus, the processing from step S8A to step S11A of the first embodiment described in Figure 3 described above is performed. Normal reflex measurement (provisional measurement, actual measurement) is performed so that the eye refractivity of the subject eye E can be measured with high precision.

On the other hand, when the subject eye E has nystagmus or is a subject eye of a restless child, even performing auto-alignment a predetermined number of times does not set the current position of the measurement head 18 within the aforementioned allowable range. In this case, the second determining unit 56 determines satisfaction of the shift flag (NO in step S23 and YES in step S24). Thus, the operation mode for the auto-alignment and reflex measurement is switched from the normal mode to the quick mode (step S28).

Then, the quick-mode controlling unit 64 controls each optical system of the measurement head 18, the alignment controlling unit 58, and the ocular characteristic calculating unit 66 so as to perform quick mode measurement processing (step S29, corresponding to the quick mode controlling step of the presently disclosed subject matter). Thus, the processing from step S7B to step S8B of the first embodiment described in Figure 3 described above is performed. Thus, even from the subject eye E having nystagmus or the subject eye of a restless child, the eye refractivity of the subject eye E can be measured.

In this way, also according to the third embodiment, automatic selection processing for the operation mode in step S22 to step S25 is performed so that auto-alignment and reflex measurement can be performed automatically in the operation mode suitable for the subject eye E (subject). As a result, the same effect as that of each of the aforementioned embodiments can be obtained.

The second determining unit 56 of the third embodiment determines the presence of satisfaction of the shift flag based on whether or not the number of times of auto-alignment in the normal mode reaches a predetermined number of times, which is determined in advance, but the method for determining the presence of satisfaction of the shift flag is not particularly limited. For example, every time the relative position detecting unit 52 detects a relative position of the subject eye E, the second determining unit 56 performs processing of calculating a distance (the absolute value of XYZ coordinates or a root mean square of XYZ coordinates) from the measurement head 18 to the subject eye E (which may be the aforementioned allowable range) and processing of counting the number of threshold excesses that is the number of times that the distance exceeds a predetermined threshold value. Then, the second determining unit 56 determines the presence of satisfaction of the shift flag based on whether or not the number of threshold excesses reaches a predetermined number (m) while n auto-alignments are being performed, where any natural numbers are defined as m and n (m < n).

Also, instead of the aforementioned determination method, every time the relative position detecting unit 52 detects a relative position of the subject eye E, the second determining unit 56 may calculate a root mean square (RMS) exhibiting a variation across n relative position detection results based on results (coordinates or a distance to the subject eye E) of any plurality of (n, where n is a natural number of 2 or higher) relative position detections by the relative position detecting unit 52.

For example, the second determining unit 56 stores a result of a first relative position detection by the relative position detecting unit 52 as R1 and stores a result of the nth relative position detection as Rn. Then, the second determining unit 56 calculates an RMS by using a mathematical expression (RMS = √{(1/n)×(∑R1^2 + R2^2 + ...)}). Here, when the subject eye E has nystagmus or the subject is a restless child, the RMS calculation result increases, and, when no particular problem occurs, the RMS calculation result decreases. Thus, based on whether or not the RMS calculation result is higher than a predetermined threshold value, the second determining unit 56 determines the presence of satisfaction of the shift flag. An RMS is calculated as a variation of the relative position detection result, but it is not particularly limited when it is a statistic value indicating a variation.

### {Fourth Embodiment}

The ophthalmic device 10 of the third embodiment performs, as the normal mode measurement processing by the normal-mode controlling unit 62, the processing from step S8A to step S11A of the first embodiment, that is, provisional alignment, detection of a relative position of the subject eye E, auto-alignment, and actual measurement. On the other hand, an ophthalmic device 10 according to a fourth embodiment performs processing similar to the automatic selection processing for an operation mode from step S22 to step S25 illustrated in Figure 7 described above after a provisional measurement and before an actual measurement. Since the ophthalmic device 10 in the fourth embodiment has the same configuration as that of the ophthalmic device 10 in the third embodiment, like references are given to those having the same function or configuration as that of each of the aforementioned embodiments, and repetitive description is omitted.

Figure 8 is a flowchart illustrating a flow of normal mode measurement processing in the ophthalmic device 10 according to the fourth embodiment.

As illustrated in Figure 8, after completion of a provisional measurement (step S8A), detection of a relative position of the subject eye E by the relative position detecting unit 34 is performed (step S30), and, based on the position detection result, the first determining unit 54 determines whether the current position of the measurement head 18 is within the aforementioned allowable range or not (step S31).

When the first determining unit 54 determines a negative result (NO in step S31), the second determining unit 56 determines whether the shift flag is satisfied or not (step S32). When the second determining unit 56 determines a negative result (NO in step S32), based on the position detection result by the relative position detecting unit 34 in step S30, the alignment controlling unit 58 drives the drive mechanism 15 so as to perform an auto-alignment in the normal mode (step S33).

Subsequently, until the first determining unit 54 determines that the current position of the measurement head 18 is within the aforementioned allowable range (YES in step S31) or the second determining unit 56 determines the satisfaction of the shift flag (YES in step S32), the repetition controlling unit 60 repeatedly performs the processing from step S30 to step S33.

When the first determining unit 54 determines that the current position of the measurement head 18 is within the allowable range (YES in step S31), the auto-alignment (repetition control) ends (step S34). Then, the normal-mode controlling unit 62 controls each optical system of the measurement head 18 and the ocular characteristic calculating unit 66 so as to perform an actual measurement in the normal mode and calculate an actual measurement value of the eye refractivity of the subject eye E (step S35).

On the other hand, when the second determining unit 56 determines satisfaction of the shift flag (YES in step S32), the operation mode for the auto-alignment and reflex measurement is switched from the normal mode to the quick mode (step S36). Then, the quick-mode controlling unit 64 controls each optical system of the measurement head 18, the alignment controlling unit 58, and the ocular characteristic calculating unit 66 so as to perform auto-alignment in the quick mode (step S37) and then perform an actual measurement (fogging skipped) (step S38).

In this way, according to the fourth embodiment, also between a provisional measurement and an actual measurement in reflex measurement in the normal mode, automatic selection processing for the operation mode in step S30 to step S33 is performed so that the actual measurement can be performed automatically in the operation mode suitable for the subject eye E (subject). As a result, the same effect as that of each of the aforementioned embodiments can be obtained.

### {Others}

While, according to each of the aforementioned embodiments, the relative position detecting unit 34, 52 controls the alignment optical system 26 and observation optical system 24 so as to detect a relative position of the subject eye E relative to the measurement head 18, the method for detecting the relative position is not particularly limited, and the relative position may be detected by using, for example, a stereo camera. Also, a relative position in the XY directions may be detected by utilizing the observation optical system 24, and a relative position in the Z direction may be detected by utilizing another sensor.

Having described the example according to each of the aforementioned embodiments that a lower number of times of auto-alignment in the quick mode is performed compared to auto-alignment in the normal mode and the allowable range of the position of the measurement head 18 after completion of the alignment is increased, either one of them may only be performed. Furthermore, as auto-alignment in the quick mode, after the position of the measurement head 18 relative to the subject eye E is tried to reach the allowable range to some degree, driving the drive mechanism 15 is stopped, and, in this state, an operation for repeating the detection of a relative position by the relative position detecting unit 34, 52 may be performed.

While the operation mode selecting unit 42 according to the first embodiment and the second embodiment selects the quick mode as the operation mode for both of auto-alignment and reflex measurement when a predetermined condition (NO in step S5 or YES in step S5B) is satisfied, the presently disclosed subject matter is not limited thereto. For example, when the aforementioned predetermined condition is satisfied, the operation mode selecting unit 42 selects the normal mode as one operation mode for auto-alignment and reflex measurement and the quick mode as the other operation mode.

Also, in the third embodiment and the fourth embodiment, when the second determining unit 56 determines satisfaction of the shift flag, auto-alignment and reflex measurement are performed in the quick mode, but any one of them may be performed in the normal mode, and the other may be performed in the quick mode.

Having described each of the aforementioned embodiments with reference to the ophthalmic device 10 that measures eye refractivity of the subject eye E as an example, the presently disclosed subject matter is also applicable to an ophthalmic device that acquires various kinds of ocular characteristics (such as an intraocular pressure, the number of corneal endothelial cells, a fundus observation image, a tomographic image) other than the eye refractivity. Also, the presently disclosed subject matter is applicable to a multifunction device that acquires multiple kinds of ocular characteristics of the subject eye E, and, in this case, the method described according to each of the aforementioned embodiments is executed every time ocular characteristics different from each other are acquired, that is, every time auto-alignment is performed.

### {Reference Signs List}

10 ophthalmic device
12 base
13 face receiving unit
13a jaw receiver
13b forehead protector
14 frame
15 drive mechanism
16 operation lever
17 monitor
18 measurement head
20 control device
22 fixation target projecting optical system
24 observation optical system
26 alignment optical system
28 pattern light-projecting optical system
30 measuring optical system
32 step
34 relative position detecting unit
36 provisional alignment controlling unit
38 re-detecting unit
40 determining unit
41 repetition controlling unit
42 operation mode selecting unit
44 alignment controlling unit
46 measurement controlling unit
48 ocular characteristic calculating unit
50 control device
52 relative position detecting unit
54 first determining unit
56 second determining unit
58 alignment controlling unit
60 repetition controlling unit
62 normal-mode controlling unit
64 quick-mode controlling unit
66 ocular characteristic calculating unit
E subject eye

## Claims

1. An ophthalmic device, comprising:
an ocular characteristic acquiring unit configured to acquire an ocular characteristic of a subject eye;
a relatively-moving unit configured to relatively move the ocular characteristic acquiring unit relative to the subject eye;
a detecting unit configured to detect a relative position of the subject eye relative to the ocular characteristic acquiring unit;
a provisional alignment controlling unit configured to drive the relatively-moving unit based on a detection result by the detecting unit and perform provisional alignment of the ocular characteristic acquiring unit relative to the subject eye;
a re-detecting unit configured to repeatedly detect the relative position by the detecting unit after completion of the provisional alignment;
a determining unit configured to determine whether the ocular characteristic acquiring unit has been brought closer to the subject eye by a distance greater than or equal to a predetermined threshold value by the provisional alignment based on the detection result by the detecting unit before and after the provisional alignment;
an operation mode selecting unit configured to select either one of a normal mode and a quick mode that is simpler than the normal mode as an operation mode for alignment of the ocular characteristic acquiring unit relative to the subject eye and acquisition of the ocular characteristic by the ocular characteristic acquiring unit based on a determination result by the determining unit;
an alignment controlling unit configured to drive the relatively-moving unit based on the detection result by the detecting unit after completion of the provisional alignment and perform the alignment in the operation mode selected by the operation mode selecting unit; and
a measurement controlling unit configured to cause acquisition of the ocular characteristic to be performed by the ocular characteristic acquiring unit in the operation mode selected by the operation mode selecting unit after completion of the alignment,
wherein the operation mode selecting unit performs first selection processing that selects the normal mode as the operation mode for both of the alignment and the acquisition of the ocular characteristic when the determining unit determines that the ocular characteristic acquiring unit has been brought closer to the subject eye by a distance greater than or equal to the threshold value and selects the quick mode as the operation mode for at least one of the alignment and the acquisition of the ocular characteristic when the determining unit determines that the ocular characteristic acquiring unit has not been brought closer to the subject eye by a distance greater than or equal to the threshold value.

2. The ophthalmic device according to claim 1,
wherein the provisional alignment controlling unit can perform the provisional alignment based on the detection result by the detecting unit after completion of the provisional alignment,
the ophthalmic device comprises a repetition controlling unit configured to perform repetition control that causes the provisional alignment controlling unit, the re-detecting unit, and the determining unit to repeatedly operate one or more times after a first determination by the determining unit, and
when the repetition control is performed, the operation mode selecting unit performs, instead of the first selection processing, second selection processing that selects the quick mode as the operation mode for at least one of the alignment and the acquisition of the ocular characteristic when the number of determinations that the determining unit determines that the ocular characteristic acquiring unit has not been brought closer to the subject eye by a distance greater than or equal to the threshold value has reached a predetermined number of times that is determined in advance, and selects the normal mode as the operation mode for both the alignment and the acquisition of the ocular characteristic when the number of determinations has not reached the predetermined number of times and the number of repetition controls has reached a predetermined upper limit number of times.

3. An ophthalmic device, comprising:
an ocular characteristic acquiring unit configured to acquire an ocular characteristic of a subject eye;
a relatively-moving unit configured to relatively move the ocular characteristic acquiring unit relative to the subject eye;
a detecting unit configured to detect a relative position of the subject eye relative to the ocular characteristic acquiring unit;
a first determining unit configured to determine whether the position of the ocular characteristic acquiring unit is within an allowable range of an alignment position of the ocular characteristic acquiring unit relative to the subject eye based on a detection result of the detecting unit;
a second determining unit configured to, when the first determining unit determines a negative result, determine whether or not a shift flag is satisfied for shifting the operation mode for at least one of alignment of the ocular characteristic acquiring unit relative to the subject eye by the relatively-moving unit and acquisition of the ocular characteristic by the ocular characteristic acquiring unit from a normal mode to a quick mode that is simpler than the normal mode;
an alignment controlling unit configured to, when the second determining unit determines a negative result, drive the relatively-moving unit and perform the alignment in the normal mode based on the detection result by the detecting unit;
a repetition controlling unit configured to cause the detecting unit, the first determining unit, the second determining unit, and the alignment controlling unit to repeatedly operate until the first determining unit determines that the position is within the allowable range or until the second determining unit determines that the shift flag is satisfied;
a normal-mode controlling unit configured to, when the first determining unit determines that the position is within the allowable range, cause the ocular characteristic acquiring unit to perform acquisition of the ocular characteristic in the normal mode; and
a quick-mode controlling unit configured to, when the second determining unit determines that the shift flag is satisfied, cause at least one of the alignment by the alignment controlling unit and the acquisition of the ocular characteristic by the ocular characteristic acquiring unit to be performed in the quick mode.

4. The ophthalmic device according to claim 3, wherein the second determining unit counts the number of times of the alignment by the alignment controlling unit and, based on whether or not the number of times of the alignment has reached a predetermined number of times that is determined in advance, determines the presence of the satisfaction of the shift flag.

5. The ophthalmic device according to claim 3, wherein the second determining unit performs processing of detecting a distance from the ocular characteristic acquiring unit to the subject eye every time the detecting unit detects the relative position and processing of counting the number of times that the distance exceeds the predetermined threshold value and determines the presence of the satisfaction of the shift flag based on whether or not the number of times the distance exceeds the threshold value has reached a predetermined number of times that is determined in advance.

6. The ophthalmic device according to claim 3, wherein the second determining unit calculates a variation across a plurality of detection results of the relative position over a plurality of detection results every time the detecting unit detects the relative position and determines the presence of the satisfaction of the shift flag based on whether the variation is higher than a predetermined threshold value or not.

7. A control method for an ophthalmic device, comprising:
a detecting step of detecting a relative position of a subject eye relative to an ocular characteristic acquiring unit configured to acquire an ocular characteristic of the subject eye;
a provisional alignment step of relatively moving the ocular characteristic acquiring unit relative to the subject eye based on a detection result by the detecting step and performing provisional alignment of the ocular characteristic acquiring unit relative to the subject eye;
a re-detecting step of repeatedly performing the detecting step after completion of the provisional alignment;
a determining step of determining whether the ocular characteristic acquiring unit has been brought closer to the subject eye by a distance greater than or equal to a predetermined threshold value by the provisional alignment based on the detection result by the detecting step before and after the provisional alignment;
an operation mode selecting step of selecting either one of a normal mode and a quick mode that is simpler than the normal mode as an operation mode for alignment of the ocular characteristic acquiring unit relative to the subject eye and acquisition of the ocular characteristic by the ocular characteristic acquiring unit based on a determination result by the determining step;
an alignment step of relatively moving the ocular characteristic acquiring unit relative to the subject eye based on the detection result by the detecting step after completion of the provisional alignment and performing the alignment in the operation mode selected by the operation mode selecting step; and
an ocular characteristic acquiring step of causing acquisition of the ocular characteristic to be performed by the ocular characteristic acquiring unit in the operation mode selected by the operation mode selecting step after completion of the alignment,
wherein the operation mode selecting step performs first selection processing that selects the normal mode as the operation mode for both of the alignment and the acquisition of the ocular characteristic when the determining step determines that the ocular characteristic acquiring unit has been brought closer to the subject eye by a distance greater than or equal to the threshold value and selects the quick mode as the operation mode for at least one of the alignment and the acquisition of the ocular characteristic when the determining step determines that the ocular characteristic acquiring unit has not been brought closer to the subject eye by a distance greater than or equal to the threshold value.

8. A control method for an ophthalmic device, comprising:
a detecting step of detecting a relative position of a subject eye relative to the ocular characteristic acquiring unit configured to acquire an ocular characteristic of the subject eye;
a first determining step of determining whether the relative position detected by the detecting step is within an allowable range of an alignment position of the ocular characteristic acquiring unit relative to the subject eye;
a second determining step of, when the first determining step determines a negative result, determining whether a shift flag is satisfied for shifting the operation mode for at least one of alignment of the ocular characteristic acquiring unit relative to the subject eye and acquisition of the ocular characteristic by the ocular characteristic acquiring unit from a normal mode to a quick mode that is simpler than the normal mode;
an alignment step of relatively moving the ocular characteristic acquiring unit relative to the subject eye based on a detection result by the detecting step when the second determining step determines a negative result and performing the alignment in the normal mode; and
a repeating step of repeatedly performing the detecting step, the first determining step, the second determining step, and the alignment step until the first determining step determines that the position is within the allowable range or until the second determining step determines that the shift flag is satisfied;
a normal-mode controlling step of, when the first determining step determines that the position is within the allowable range, causing the ocular characteristic acquiring unit to perform acquisition of the ocular characteristic in the normal mode; and
a quick-mode controlling step of, when the second determining step determines that the shift flag is satisfied, causing at least one of the alignment and the acquisition of the ocular characteristic by the ocular characteristic acquiring unit to be performed in the quick mode.
